# EUROPEAN PATENT APPLICATION

(11) **EP 3 260 169 A2**
(43) Date of publication of application: **27.12.2017**
(21) Application number: 17177691.7
(22) Date of filing: 23.06.2017
(51) Int. Cl.: A61N 5/10, G21F 3/00, G21F 1/12, G21K 1/02, B23K 26/38

(54) **METHOD FOR PROVIDING A PATIENT SPECIFIC RADIATION SHIELD FOR RADIATION THERAPY**

(30) Priority: 23.06.2016 GB 201610950
(71) Applicant: Ariane Medical Systems Limited, Alfreton, Derbyshire DE55 7JQ (GB)
(72) Inventor: SPANSWICK, Christopher Keith, Alfreton, Derbyshire DE55 7JQ (GB); SPANSWICK, Keith Albert, Alfreton, Derbyshire DE55 7JQ (GB)
(74) Representative: HGF Limited

(57) **Abstract**

Embodiments of the present invention provide a patient specific radiation shield for use with a radiotherapy applicator, to prevent exposure of healthy tissue to radiation. A method of providing such comprises: creating a record of an area, which corresponds to a target area to be treated by radiotherapy; cutting the radiation shield from a shield material, comprising cutting an aperture into the shield material, wherein the aperture shape corresponds to the record of the area.

## Description

### TECHNICAL FIELD

Aspects of the invention relate generally to a method for making a radiation shield, to a radiation shield and to a system for making a radiation shield. More specifically, aspects of the invention relate to a method for providing a patient specific radiation shield, a treatment specific applicator for use in radiation therapy, and a system for producing a patient specific radiation shield.

### BACKGROUND

Radiotherapy is a treatment that uses carefully measured transmission of radiation to induce cell death in a tissue. Typically in radiotherapy, and specifically in soft x-ray therapy, a treatment area on the patient is first identified by a clinician. Radiation is then generated by a radiation source and directed to the identified target area. Because radiation induces cellular death, it is important to shield healthy tissue from the incident radiation.

For some x-ray therapies an applicator may optionally be used in combination with the x-ray source. An applicator is an apparatus that provides a fixed distance between the patient and the x-ray source and also provides collimation of the incident radiation. The applicator is usually of tubular construction, often comprising stainless steel or a similar material. The clinician will typically position the applicator over the target area of the patient and clamp the applicator in position. The radiation source is then connected to the applicator and used to safely deliver the required dose of radiation to the target area.

To reduce the amount of healthy tissue that is exposed to radiation, the clinician will select an applicator with a diameter that most closely matches the maximum width of the target area. Additionally, the clinician may also apply pieces of metallic foil or similar against the patient to mask the healthy tissue from the incident radiation. The foils are usually placed before positioning and clamping the applicator. With this method, invariably some small areas of healthy tissue may still be subject to an unwanted dose of radiation. Furthermore, the process, whilst fairly quick to set up, could be further improved.

It is an object of embodiments of the invention to at least mitigate one or more of the problems of the prior art, or to provide improvements generally.

### SUMMARY OF THE INVENTION

In an aspect, there is a method for providing a patient specific radiation shield for use with a radiotherapy applicator, comprising: creating a record of an area, which corresponds to a target area to be treated by radiotherapy; cutting the radiation shield from a shield material, comprising cutting an aperture into the shield material, wherein the aperture shape corresponds to the record of the area. This may provide a straight-forward, rapid, and low cost method of producing a patient specific radiation shield, to further minimise the exposure to radiation of healthy tissue during radiotherapy.

In embodiments, the patient specific radiation shield for use with a brachytherapy applicator.

In an embodiment, the record of the area comprises an electronic record. An electronic record may be any record capable of being stored on electronic storage media. Or, an electronic record comprises, but is not limited to, a digital record, photograph, an image derived by any known method of medical imaging, or any file type containing data relating to volume, area, surface, shape or position for example. Use of an electronic, or digital record, may provide additional improvements in accuracy when providing a patient specific radiation shield.

In an embodiment, the aperture is cut using a computer controlled cutting device. This may provide additional improvements in accuracy for providing a patient specific radiation shield.

In an embodiment, the aperture is cut using a digital stencil cutter. This may provide additional improvements in accuracy providing a patient specific radiation shield. This may also provide a low-cost and quick method of providing a patient specific radiation shield.

In an embodiment, creating a record of an area comprises: creating an image of an area to be treated; and creating a record of an area from the image of the area to be treated. This may provide additional improvements in accuracy for providing a patient specific radiation shield.

In an embodiment, creating a record of an area may comprise taking a photo of an area to be treated.

In an embodiment, creating the record comprises: drawing, an outline around, or an area (or mask) over, the area to be treated on the image. This may provide additional improvements in accuracy for providing a patient specific radiation shield. The area or outline may be drawn manually using image processing software, or it may be drawn automatically using software, or a combination of both, for example.

In an embodiment, a reference marker or phantom is contained within the field of view of the image, and a dimension of the reference marker is used to calibrate the scale of the image. This may provide additional improvements in accuracy and speed for providing a patient specific radiation shield.

In an embodiment, the radiation shield may comprise two or more layers of a shield material, wherein an aperture may be cut into one or more layers of shield material. This may provide further improved adaptability or accuracy of the radiation shield.

In an embodiment, two or more of the layers of the shield material may comprise at least one aperture having a different shape from another aperture. This may allow further improved adaptability of control of the amount of radiation delivered to different regions of the treatment area. In an embodiment, the two or more of the layers of the shield material may each comprise an apertures where one aperture is a reduced scale version of the other aperture.

In an embodiment, the method may comprise: ablating a portion of the radiation shield to reduce the thickness of at least part of the shield material. This may provide additional improvements in accuracy of the patient specific radiation shield, and may allow further improvements in control of the amount of radiation delivered to different regions of the treatment area.

In an aspect, there is provided a method of producing a patient specific applicator for use in radiotherapy, comprising a method for producing a patient-specific radiation shield of the previous aspect, wherein the radiation shield is placed at the distal end of a radiotherapy applicator. This may provide additional improvements to the applicator generally.

In an embodiment, the applicator may be a brachytherapy applicator.

In an embodiment, an end-cap may be applied to the distal end of the radiotherapy applicator to retain the radiation shield in position. This may provide a quick and low cost method for retaining the radiation shield to the applicator. The end cap may also be disposable, which may improve infection control.

In an embodiment, the distal end of the radiotherapy applicator may comprise at least one window. This may provide the user with improved ease of locating the applicator against the target area.

In an aspect, there is provided a treatment specific applicator for use in radiation therapy, comprising: a radiotherapy applicator, the applicator comprising a patient-contacting distal end and a radiation source contacting proximal end, the applicator having a length to define a distance between the patient and the radiation source; and a radiation shield of an aspect of the invention as described herein, the shield being positioned at the distal end of the applicator, and comprising an aperture, wherein the shape of the aperture corresponds to the shape of a target area to be treated by radiotherapy. This may provide an applicator which reduces the exposure of healthy tissue to radiation during radiotherapy.

In an embodiment, the radiation shield may comprise two or more layers of a shield material, wherein an aperture is cut into one or more layers of shield material. This may provide further improved adaptability or accuracy of the radiation shield.

In an embodiment, two or more of the layers of shield material may comprise an aperture having a different shape from another aperture. This may provide further improved adaptability or accuracy of the radiation shield.

In an embodiment, the shield material may comprise a metal sheet. This may provide good attenuation of radiation and can be cut easily. In an embodiment, the shield material may comprise a sheet of a polymer-metal material. This may provide further improvements for attenuating of radiation and ease of cutting. In an embodiment, the radiation shield may comprise a sheet of lead-rubber material. This may provide further improvements of attenuating radiation and/or improved ease of cutting.

In an aspect or embodiment, an applicator may comprise an end-cap applied to the distal end of the applicator. In an embodiment, the treatment specific applicator may comprise an end-cap which retains the radiation shield against the distal end of the applicator. In an embodiment, the end-cap may be optically transparent. In an embodiment, the end-cap may comprise a shape which conforms to end of the applicator, or may conform to a recess in the distal end of the applicator. In an embodiment, the radiation shield may have a generally circular perimeter with a diameter corresponding to the internal diameter of the distal end of the applicator. In an embodiment, the treatment specific applicator may comprise a window that permits visual access to the radiation shield. These features may provide further improvements to the ease of use of the applicator.

In an embodiment, the end-cap may comprise a biocompatible surface. In an embodiment, the end-cap may comprise a sterile surface, and/or a sterilisable surface, and/or a disinfectable surface. These features may provide an improved patient contacting surface, and therefore improved patient compatibility.

In an aspect, there is provided a system for producing a patient specific radiation shield according to any method described herein, the system may comprise: an image forming device configured to create an image of a treatment area; an image processing device configured to create a record of an area to be treated; and a computer controlled cutter configured to cut a radiation shield from a shield material, the radiation shield comprising an aperture having a shape that corresponds to the record. This may provide a system capable of producing a patent specific radiation shield, with improved ease of use and low cost. In embodiments, the system may further comprise an applicator and a radiation source.

In an embodiment, the image forming device may be a digital camera.

In an embodiment, a reference marker, or if non-visible radiation is used, a phantom is contained within the field of view of the image, and a dimension of the reference marker is used to calibrate the scale of the image. This may further improve accuracy and ease of use. In an embodiment, a reference maker may be used to calibrate the colour of the image. In an embodiment, the same reference marker may be used to calibrate both colour and scale of the image.

The preceding summary is provided for purposes of summarizing some embodiments to provide a basic understanding of aspects of the subject matter described herein. Accordingly, the above-described features are merely examples and should not be construed to narrow the scope or spirit of the subject matter described herein in any way. Moreover, the above and/or proceeding embodiments may be combined in any suitable combination to provide further embodiments. Other features, aspects, and advantages of the subject matter described herein will become apparent from the following Detailed Description, Figures, and Claims.

### BRIEF DESCRIPTION OF THE DRAWINGS

Aspects, features and advantages of embodiments of the present disclosure will become apparent from the following description of embodiments in reference to the appended drawings in which like numerals denote like elements.
Figure 1 shows an aspect of an embodiment of the invention;
Figure 2 shows an aspect of an embodiment of the invention;
Figure 3 shows an embodiment of the invention; and
Figure 4 shows an embodiment of the invention in use.

### DETAILED DESCRIPTION

It is to be understood that the method, shield, applicator and system are not limited to the details of construction or process steps set forth in the following description. It will be apparent to those skilled in the art having the benefit of the present disclosure that the invention is capable of other embodiments and of being practiced or being carried out in various ways.

As used herein, the term **"radiation therapy"** includes any therapy involving the transmission of energy. Radiation therapy may include, but is not limited to, brachytherapy, contact x-ray therapy/electronic brachytherapy, near or close x-ray therapy, external beam radiotherapy, stereotactic radiosurgery and intraoperative radiotherapy. Brachytherapy may refer to radiation therapy where the source is close to or in contact with the target tissue.

As used herein, the term "**treatment area**" includes any tissue that is to be treated by radiation therapy. This may include, but is not limited to, tumours and other cancerous tissues.

As used herein, the term "**patient specific**" refers to apparatus adapted to the treatment specific to a patient.

As used herein, the term "**radiation shield**" refers to an object for controlling or restricting transmission of radiation to a patient during radiation therapy. In particular, it may refer to a screen or mask used to control radiation delivered to an area on a patient. The radiation shield may be configured by choosing its layers, thickness thereof, or material attenuation properties to provide radiation attenuation appropriate to the clinical situation in which it is to be used. Optionally, the radiation shield may be capable of attenuating greater than 30%, or greater than 50%, or greater than 70%, or greater than 90%, or greater than 95%, or greater than 98% or greater than 99.5%, or greater than 99.95% of the incident radiation for the application for which it is to be used. The shield may comprise multiple layers of a shield material, in which case, the attenuation of the radiation shield is considered to be the overall attenuation of all of the layers. Attenuation may be determined by using a radiation dose meter, or by applying the beer-lambert law.

As used herein, the term "**aperture**" includes any region that increases permittivity to incident radiation. Aperture may include, but is not limited to, holes, slits and gratings.

As used herein, the term "**applicator**" includes any apparatus that extends from a source of radiation to treatment area to control delivery of radiation to the target area on the patient. An applicator typically provides a fixed distance between the patient and the radiation source to provide control of the radiation dose delivered to the patient. The applicator may also provide collimation of the incident radiation. Conventional applicators are usually of tubular construction, with a distal end (patient facing end) provided in different diameters to fit the target area, however, they need not be limited thereto. In embodiments, the term applicator may specifically refer to an applicator for brachytherapy.

As used herein, the term "**shield material**" includes any material capable of attenuating the incident radiation from a radiation source for radiation therapy. Optionally, a shield material may be any material appropriate for attenuating radiation in the clinical situation for which it is to be used, with an attenuation of greater than 30% of the incident radiation, or greater than 50%, or greater than 70%, or greater than 90%, or greater than 95%, or greater than 98% or greater than 99.5%, or greater than 99.95%. For example a shield material may include, but is not limited to, metal-polymer materials such as lead-rubber, or materials comprising metal sheets or foils, or ceramics, polymers and natural materials. A shield material may have any thickness, or may have a thickness of from 0.05 mm to 5 mm, or from 0.1 mm to 3 mm, or from 0.25 mm to 1 mm or a range with any combination of the aforesaid values. It follows that a shield material of a certain thickness and material type, suitable for low energy x-rays, may not necessarily be suitable for high energy x-rays for example.

As used herein, the term "**cutting**" includes any process for removing or separating material. Cutting may include, but is not limited to, mechanical cutting including, for example, cutting by use of a stencil cutter, milling tool, knife or saw; a water cutter; a laser cutter; a plasma cutter or an ultrasound cutter.

As used herein, the term "**ablation**" includes any process for removing material from a surface.

As used herein, the term "**treatment specific applicator**" refers to an applicator configured to the treatment requirements of an individual patient.

As used herein, the term "**computer controlled cutting device**" includes any device wherein mode of cutting is determined via computer control. This may include, but is not limited to, a digital stencil cutter, or any cutting tool apparatus on an X-Y table/gantry or computer controlled arm.

As used herein, the term "**image forming device**" includes any apparatus capable of forming an image. This may include, but is not limited to, an optical camera, an infrared camera, an ultrasound scanner, or any imaging method used in medical diagnostics.

As used herein, the term "**image processing device**" includes any apparatus capable of receiving and processing an image. This may include, but is not limited to a computer comprising image processing software.

As used herein "**user**" is intended to refer to a medical practitioner, end user or other user associated therewith.

As used herein, the term "**external perimeter**" refers to the outermost edge, which defines the external shape of an object.

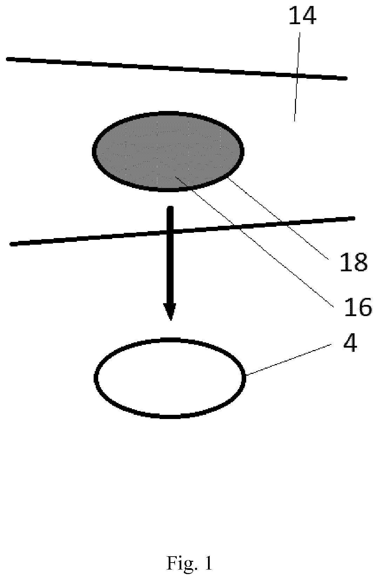

Referring to figure 1, an aspect of an embodiment is shown. In figure 1, a patient 14 having a tissue that requires treatment by radiotherapy 16 is shown. An area around the tissue may be identified as an area to be treated by radiotherapy 18, this area may also be known herein as a target area. A record 4 of the target area 20 is formed, where the record 4 is a record of the shape of the target area 18. The record may be, for example, a physical template having the same or similar shape as the target area, or it may comprise an electronic record in the form of data. This may comprise, for example, creating an electronic or digital record, such as a photograph, and digitally creating an image thereon, wherein the shape is the same, similar to, or contains information that relates to the target area.

The record may be used to form a radiation shield. The radiation shield may be formed, for example, by first cutting an aperture into a sheet of shield material. Where the record comprises, for example a physical template, this may involve simply placing the template over a sheet of a shield material and cutting around the template. Alternatively, where the record may comprise an electronic or digital record, this may be converted by a processor comprising appropriate software, to an input (e.g. a numerical control) for a computer-controlled cutting device. The computer controlled cutting device may then cut an aperture having a shape that corresponds to the shape recorded by the record 4. Forming the radiation shield may also comprise cutting the desired external perimeter 28 into the shield material, and optionally cutting or separating the radiation shield from the piece or sheet of shield material. The record may contain additional data corresponding to the external perimeter, to control the cutting from the shield material. Alternatively, the sheet of shield material may be formed to correspond to the desired external perimeter prior to cutting of the aperture.

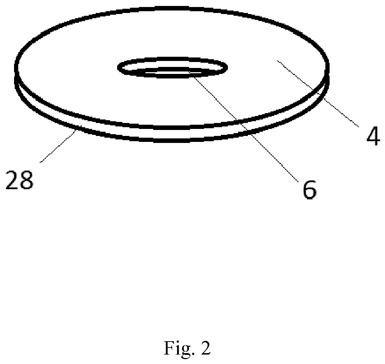

Referring to figure 2, a radiation shield 2 of an embodiment is shown. The radiation shield comprises an external perimeter 28, and an aperture 6 having a shape that corresponds to the record of the treatment area. The radiation shield may be applied to an applicator or to the target area, so that incident radiation is delivered only to the target area and not to surrounding healthy tissue.

Figure 2 shows a radiation shield comprising a single layer of a shield material. The shield material may however comprise two or more layers (not shown). These may be layers of the same material or layers of different materials. The layers may be cut in the same cutting step or each layer may be cut separately and subsequently combined. The layers may be combined, by any of the following non-limiting examples: using adhesion, being pressed together or retained in position using friction when applied to an applicator. Each layer of a radiation shield may be cut to have the same shaped aperture or a different shaped aperture. This may be used to provide different doses of radiation to different regions of the target area. For example, the target area may comprise a region to receive a high dose and a region to receive a lower dose of radiation. A radiation shield may be formed with two layers for example, one layer of shield material comprises an aperture, and the second layer comprising a smaller aperture. When the two layers are combined the apertures may be arranged so that a clear path is present through both apertures. As well as a region around the smaller aperture where one layer of shield material with the smaller aperture may be present to partially attenuate the incident radiation, and a region with two layers that fully attenuates the incident radiation. The two layers may be the same or they may comprise different materials or thicknesses, selected by the clinician to provide the appropriate attenuation. Different layers may also be selected for their different attenuation properties, for example, some may be selected for its attenuation properties, and another for its beam hardening properties.

A radiation shield may also be formed using ablation, either in place of, or in combination with cutting. For example, a radiation shield, including the aperture, may be formed by using ablation to isolate it from the sheet of solid material. Alternatively, ablation may be used in combination with cutting, either to form the aperture or separate the shield from the shield material. Alternatively, ablation may be used to reduce the thickness of the radiation shield to locally reduce attenuation. For example, where the aperture comprises a metal-foil polymer laminate, ablation (or alternatively cutting) may be used to form an aperture by removing the foil from the polymer layer, whilst optionally retaining a polymer layer intact. This would provide an aperture for radiation to pass without providing a visible hole through the radiation shield. Ablation may also be used, for example, to locally reduce the thickness of the shield material to create regions on the radiation shield that attenuates the incident radiation to different extents, to provide different doses of radiation to the target area. This may be achieved, for example, by creating regions on the record, which may be used to perform ablation to different extent using a computer controlled ablation device.

Lead-rubber may be used as the shield material, optionally as a single layer, as multiple layers, or in combination with a layer (or layers) of other materials. Lead-rubber may be beneficial because it provides good radiation attenuation characteristics for many types of radiotherapy radiation types. Lead-rubber is well suited to attenuating radiation from near/close x-ray therapy/brachytherapy, and contact x-ray therapy/brachytherapy. Lead-rubber can be cut easily by hand and by known computer controlled cutting methods. Lead-rubber is well suited to being cut by a computer controlled stencil cutter. The thickness of lead rubber may include the following ranges from 0.05 mm to 5 mm, or from 0.1 mm to 3 mm, or from 0.25 mm to 1 mm or a range with any combination of the aforesaid values.

As used herein, the term "**lead-rubber**" includes any material comprising lead and a rubber. This includes, but is not limited to, all known lead-rubber composites, particularly laminates comprising at least a layer of lead and at least a layer of rubber or any non-laminate lead-rubber materials.

An exemplary lead-rubber includes, but is not limited to 0.25 mm Lear-rubber sheet by ProtecX Medical Ltd, United Kingdom.

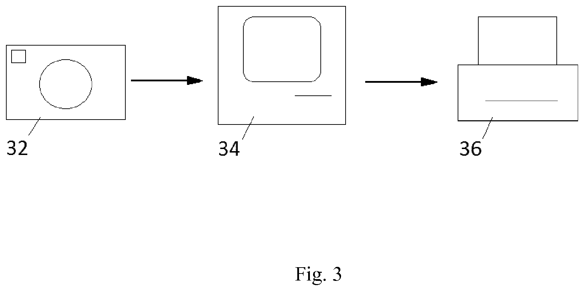

Referring to figure 3, a system 30 of an embodiment of the invention is shown. The system comprises an image forming device 32 (for example, a digital camera), an image processing device 34 (for example, a computer comprising image processing software), and a computer controlled cutter 36 (for example, a computer-controlled stencil cutter).

The image forming device may be used to create an image of a treatment area, for example where the image forming device is a digital camera this may be by taking a photo of the treatment area.

The image processing device 32 may be used to create a record of the treatment area. Where the image is a photo, this may be by, for example, manually or automatically drawing a shape over or an outline around the image of the treatment area using image processing software with the image processing device. The drawn shape or outline may then comprise a record that corresponds to the treatment area. Optionally, a shape or outline corresponding to the external perimeter of a radiation shield may also be included in the record. The record may then be converted by the image processing device 34, using appropriate software to a format that can be exported to control the computer-controlled cutter 36, to cut a radiation shield 2 from a shield material. The radiation shield 2 that is cut may comprise an aperture 6 having a shape that corresponds to the record 4, and therefore to the treatment area. As used herein, the term "**corresponds**" includes any relationship whereby the record is used to provide an aperture in a shape that matches the treatment area. This may include, but is not limited to being an exact match, or a scaled version thereof, or comprising values that can be converted/processed to produce an aperture that is an exact match of, or a scaled version of the target area.

Creating a record may comprise any of, but not be limited to any of the following processes: binarising or thresholding the image, applying edge detection algorithms, applying templates or using sphere fitting.

In some embodiments it may be necessary to calibrate the image so that it contains data allowing the image or items represented within the image to have known dimensions. This may be achieved, for example, by using a marker or phantom with a known dimension, in the field of view when the image is created. For example, when creating a photograph, a circular marker of known radius may be placed near the area to be treated. A scale may then be applied to the photograph so that the radius of the marker on the image is equivalent to the known radius of the marker. This may be performed by image processing software, for example. Alternatively, the camera may be arranged a predetermined distance from the patient so that an image of known dimensions is created. The colour of a marker may also be used to calibrate the colour or adjust the colour balance of an image. This may provide benefits when using image processing algorithms such as edge detection to define the area.

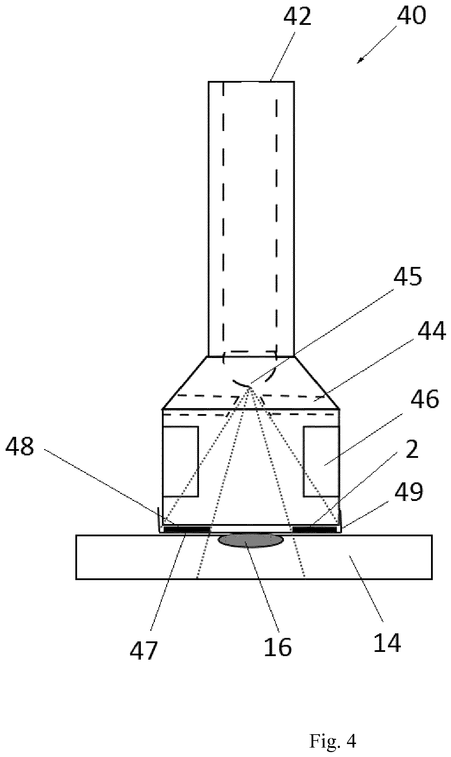

Referring to figure 4, an applicator 40 is shown. The applicator may comprise an elongate tubular body with an opening to an internal diameter at a proximal end 42 that corresponds to an external diameter of an x-ray source 45. Thereby allowing the applicator to be attached thereto. The cross section of the applicator may be circular, oval, polygonal, square, or any other hollow shape. The applicator 40 may comprise a collimator 44 to minimise penumbra effects at the distal end of the applicator. The applicator may also comprise a radiation shield 2, which may be positioned at or close to the distal end of the applicator. The radiation shield 2 may comprise an aperture that defines the area to receive treatment by the incident radiation. The radiation shield may be patient specific as described previously. Alternatively, the radiation shield may not be patient specific, for example, the radiation shield may comprise a circular aperture with a predetermined diameter. Alternatively, the radiation shield may be integrated into the internal wall of the applicator. The applicator may also comprise at least one window 46 near the distal end 48 to allow a user to view the position of the aperture in the radiation shield when it is placed against a patient. The collimator may be positioned to prevent incident radiation passing through the window 46.

The applicator may be used by positioning against a patient 14 so that the aperture of the radiation shield 2 is aligned with the area to be treated. To facilitate this, the applicator may comprise windows 46 to allow the user to view the area to be treated 16 through the aperture. The applicator may be clamped to retain its position, and the radiation source inserted into the internal diameter of the applicator 40. Alternatively, the radiation source may be inserted into the applicator 40 prior to positioning, so that the applicator and source may be positioned simultaneously. Once in position, radiation from the radiation source 45 is directed towards the radiation shield 2. The radiation shield 2 ensures that radiation passes through the aperture 6 only, so that radiation is delivered to the target area 16 and not to surrounding healthy tissue.

Applicator may be made from, but not limited to: metals, including steel or stainless steel, or most rigid polymers. The applicator may be of overall tubular construction, or of square, oval or triangular cross-section, for example. The applicator may comprise a wider distal end than proximal end. The applicator may comprise a distal end with an internal diameter of from 100 mm to 10 mm, from 70 mm to 30 mm, or from 40 mm to 50 mm and/or a range with any combination of the aforementioned end point. The applicator may comprise a distal end with an internal diameter of 80 mm, 70 mm 60 mm, 50 mm, 40 mm or 30 mm. The radiation shield may comprise an external perimeter of circular shape having a diameter to match the internal diameter of an applicator. The applicator may be compatible to work with any radiation therapy source, including, for example: Papillion 50 or Papillion + by Ariane Medical Systems, Derby, UK.

The applicator may further comprise an end-cap 49 that is placed over the distal end 48 of the applicator 40. The end-cap 49 may be retained against the applicator by using, for example, an interference fit, or it may comprise a lip or groove which corresponds with a lip or groove on the applicator (not shown), or by any other retention means known. The end-cap may be used to retain the radiation shield 2 against the distal end of the applicator 48, or retain it within the distal end of the applicator (not shown). The end-cap 49 may comprise markings on an external surface to identify an orientation of the radiation shield within to facilitate alignment of the radiation shield with the target area (not shown). The end-cap may also comprise a surface that can be sterilised or disinfected 47, preferably the patient facing surface. Alternatively the endcap may be provided with a sterile surface, for example, the endcap may be provided in a sterile container or with a sterile patch to be removed, to present a sterile surface for contacting the patient 14. Where the radiation shield 2 comprises a screen material that is a lead-rubber or a material that is undesirable to contact the patient, the endcap 49 functions as a barrier, preventing the material contacting the patient 14. The endcap 49 may also be transparent. This may permit a user to view the target area to be treated 16 through the aperture of the radiation shield via a window of the applicator to facilitate positioning of the applicator 40. The endcap they also comprise an opening to permit the user to view the area to be treated in the same manner, or may, for example, comprise a gauze, a mesh or similar. The end-cap may comprise a material that provides minimal attenuation of incident radiation, this may be, but not limited to, a polymer such as particularly a low density polymer, e.g. poly-methyl-methacrylate of low density poly ethylene. The end-cap may dimensioned to co-operate with the distal end of the applicator.

In the claims, any reference signs placed between parentheses shall not be construed as limiting the claim. The word 'comprising' does not exclude the presence of other elements or steps than those listed in a claim. Furthermore, the terms "a" or "an," as used herein, are defined as one or as more than one. Also, the use of introductory phrases such as "at least one" and "one or more" in the claims should not be construed to imply that the introduction of another claim element by the indefinite articles "a" or "an" limits any particular claim containing such introduced claim element to inventions containing only one such element, even when the same claim includes the introductory phrases "one or more" or "at least one" and indefinite articles such as "a" or "an." The same holds true for the use of definite articles. Unless stated otherwise, terms such as "first" and "second" are used to arbitrarily distinguish between the elements such terms describe. Thus, these terms are not necessarily intended to indicate temporal or other prioritization of such elements. The mere fact that certain measures are recited in mutually different claims does not indicate that a combination of these measures cannot be used to advantage.

Unless otherwise explicitly stated as incompatible, or the physics or otherwise of the embodiments, example or claims prevent such a combination, the features of the foregoing embodiments and examples, and of the following claims may be integrated together in any suitable arrangement, especially ones where there is a beneficial effect in doing so. This is not limited to only any specified benefit, and instead may arise from an "ex post facto" benefit. This is to say that the combination of features is not limited by the described forms, particularly the form (e.g. numbering) of the example(s), embodiment(s), or dependency of the claim(s). Moreover, this also applies to the phrase "in one embodiment", "according to an embodiment" and the like, which are merely a stylistic form of wording and are not to be construed as limiting the following features to a separate embodiment to all other instances of the same or similar wording. This is to say, a reference to 'an', 'one' or 'some' embodiment(s) may be a reference to any one or more, and/or all embodiments, or combination(s) thereof, disclosed. Also, similarly, the reference to "the" embodiment may not be limited to the immediately preceding embodiment.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed. The claims should not be construed to cover merely the foregoing embodiments, but also any embodiments which fall within the scope of the claims.

The foregoing description of one or more implementations provides illustration and description, but is not intended to be exhaustive or to limit the scope of the invention to the precise form disclosed. Modifications and variations are possible in light of the above teachings or may be acquired from practice of various implementations of the present disclosure.

## Claims

1. A method for providing a patient specific radiation shield (2) for use with a radiotherapy applicator, comprising:
creating a record of an area (4), which corresponds to a target area to be treated by radiotherapy;
cutting the radiation shield (2) from a shield material, comprising cutting an aperture (6) into the shield material (8), wherein the aperture shape is corresponds to the record of the area (4).

2. A method according to claim 1, wherein the record of the area (4) comprises an electronic record.

3. A method according to any of the previous claims, wherein the aperture (6) is cut using a computer controlled cutting device or a digital stencil cutter.

4. A method according to any of the previous claims, wherein creating a record of an area comprises:
creating an image of an area to be treated; and
creating a record of an area (4) from the image of an area to be treated, and optionally wherein creating a record of an area comprises:
drawing an outline around or a mask over the area to be treated (4) on the image and/or wherein a reference marker is contained within the field of view of the image, and a dimension of the reference marker is used to calibrate the scale of the image.

5. A method according to any of the previous claims wherein the radiation shield (2) comprises two or more layers of a shield material (8), and wherein an aperture (6) is cut into one or more layers of shield material (8), and optionally wherein two or more of the layers of shield material (8) comprise an aperture having a different shape (6).

6. A method according to any of the previous claims, comprising:
ablating a portion of the radiation shield (2) to reduce the thickness of at least part of the shield material (8).

7. A method according to any of the previous claims, wherein the shield material (8) comprises a metal sheet, a polymer-metal material or a sheet of a lead-rubber material.

8. A method of producing a patient specific applicator for use in radiotherapy, comprising the method of any of the previous claims, wherein the radiation shield (2) is placed at the distal end of a radiotherapy applicator (10), and optionally wherein an end-cap (12) is applied to the distal end of the radiotherapy applicator (10) which retains the radiation shield (2) in position.

9. A method according to claim 8, wherein the end-cap (12) is optically transparent and/or wherein the end-cap (12) comprises a sterilisable or a disinfectable surface.

10. A treatment specific applicator for use in radiation therapy, comprising:
a radiotherapy applicator (10), comprising a patient-contacting distal end and a radiation source contacting proximal end, the applicator having a length to define a distance between the patient and the radiation source; and
a radiation shield (2) positioned at the distal end of the applicator (10), the radiation shield (2) comprising an aperture (6), wherein the shape of the aperture (6) corresponds to the shape of a target area to be treated by radiotherapy.

11. A treatment specific applicator according to claim 10, wherein the radiation shield comprises a sheet of lead-rubber material.

12. A treatment specific applicator according to any of claims 10 or 11, comprising an end-cap configured to be applied to the distal end of the applicator, and optionally wherein the end-cap (12) is configured to retain the radiation shield against the distal end of the applicator.

13. A treatment specific applicator according to claims 12, wherein the end-cap (12) is optically transparent, and/or wherein the end-cap (12) comprises a biocompatible surface and/or wherein the end-cap (12) comprises a sterile surface, a sterilisable surface, or a disinfectable surface, and/or wherein the end cap is disposable.

14. A treatment specific applicator according to any of claims 10 to 13, wherein the applicator (10) comprises a window to permit visual access to the radiation shield (2).

15. A system for producing a patient specific radiation shield, the system comprising:
an image forming device (20) configured to create an image of a treatment area;
an image processing device (22) configured to create a record of an area to be treated; and
a computer controlled cutter (24) configured to cut a radiation shield from a shield material comprising, the radiation shield comprising an aperture having a shape that corresponds to the record.
